(19) Europäisches Patentamt
European Patent Office
Office européen des brevets

(11) Numéro de publication : **0 052 564**
**B1**

# (12) FASCICULE DE BREVET EUROPÉEN

(45) Date de publication du fascicule du brevet : **17.10.84**

(51) Int. Cl.³ : **C 07 D417/04**, A 61 K 31/425

(21) Numéro de dépôt : **81401791.9**

(22) Date de dépôt : **12.11.81**

## (54) Dérivés de l'acide thiazolyl-2 oxamique substitués en position – 4 par un radical thiényle ou furyle, leur préparation et leur utilisation comme médicaments.

(30) Priorité : **19.11.80 FR 8024608**

(43) Date de publication de la demande : **26.05.82 Bulletin 82/21**

(45) Mention de la délivrance du brevet : **17.10.84 Bulletin 84/42**

(84) Etats contractants désignés : **AT BE CH DE GB IT LI LU NL SE**

(56) Documents cités :
**EP-A- 0 006 368**
**GB-A- 1 126 134**
**GB-A- 2 023 580**
**US-A- 2 481 673**
**CHEMISCHES ZENTRALBLATT, no. 27, partie II, 3 juillet 1963**
**CHEMICAL ABSTRACTS, vol. 95, no. 13, 25 septembre 1981, page 39, abrégé 108536e COLUMBUS OHIO (US)**
**Le dossier contient des informations techniques présentées postérieurement au dépôt de la demande et ne figurant pas dans le présent fascicule.**

(73) Titulaire : **PIERRE FABRE S.A.**
**125, rue de la Faisanderie**
**F-75116 Paris (FR)**

(72) Inventeur : **Cousse, Henri**
**La Foun de Los Noblos Chemin de Lastinos**
**F-81100 Castres (FR)**
Inventeur : **Mouzin, Gilbert**
**21, rue Sainte Foy**
**F-81100 Castres (FR)**
Inventeur : **Tarayre, Jean-Pierre**
**Rue des Sports Valdurenque**
**F-81100 Castres (FR)**
Inventeur : **Bonnaud, Bernard**
**2, rue Georges Bizet**
**F-81100 Castres (FR)**
Inventeur : **Fauran, François**
**Boisset Labege**
**F-31320 Castanet Tolosan (FR)**

(74) Mandataire : **Doat, Jean-Pierre**
**PIERRE FABRE S.A. Service Propriété Industrielle 17, Avenue Jean Moulin**
**F-81106 Castres Cedex (FR)**

**EP 0 052 564 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention, réalisée au Centre de Recherches Pierre FABRE, concerne de nouveaux dérivés de l'acide thiazolyl-2 oxamique, substitués en position – 4 par un radical thienyle ou furyle, leur procédé de préparation et leur utilisation en thérapeutique, notamment dans le traitement de l'asthme allergique.

L'invention vise également les compositions pharmaceutiques contenant ces principes actifs. La présente invention concerne de nouveaux dérivés de l'acide thiazolyl-2 oxamique substitués en position – 4 par un radical thienyle ou furyle de formule générale I :

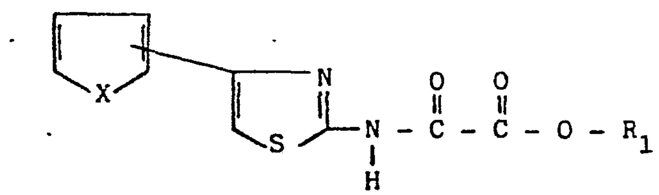

(I)

dans laquelle :

$R_1$ représente un atome d'hydrogène ou un radical alcoyle comprenant de 1 à 4 atomes de carbone et X représente un atome de soufre ou d'oxygène.

Dans la présente description, le radical alcoyle représente un groupe méthyle, éthyle, propyle, isopropyle, butyle, sec. butyle et terbutyle. Les radicaux thienyle et furyle peuvent être liés au cycle thiazole en position α ou β par rapport à l'hétéroatome oxygène ou soufre. L'invention vise également les sels avec les bases organiques ou minérales des composés de formule générale I, dans lesquels $R_1$ représente un atome d'hydrogène.

Les nouveaux composés de formule générale I peuvent être obtenus par réaction d'amino-2 thiazole de formule générale II :

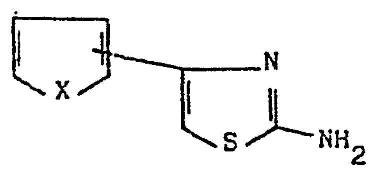

(II)

dans laquelle X possède les significations indiquées précédemment, avec un halogénure d'ester de l'acide oxalique, de préférence le chlorure ou avec un diester de l'acide oxalique et éventuellement saponification du groupe ester puis salification de l'acide obtenu par une base minérale ou organique.

Les composés chimiques nouveaux suivants et leur mode de préparation sont cités à titre d'exemples non limitatifs :

### Exemple 1

(α thienyl-4 thiazolyl-2) oxamate d'éthyle

a) α thienyl-4 amino-2 thiazole

Dans une suspension de 54 $cm^3$ (0,5 mole) d'acétyle-2 thiophène et 76,11 g (1 mole) de thiourée, on ajoute goutte à goutte 48,5 $cm^3$ (0,6 mole) de chlorure de sulfuryle.

La réaction est exothermique, le milieu réactionnel est maintenu à 110 °C sous forte agitation pendant 6 heures.

Laisser revenir à température ambiante et laver la masse cristalline par de l'éther isopropylique.

Dissoudre les cristaux obtenus dans deux litres d'eau bouillante, filtrer, et traiter la solution aqueuse par de l'ammoniaque.

Le produit cristallise, on récupère par filtration le produit brut que l'on dissout dans 300 $cm^3$ d'acétate d'éthyle et précipite par addition d'éther isopropylique.

Le produit est récupéré après séchage avec un rendement de 60 % sous forme de cristaux orangés (Point de fusion : 132 °C).

b) (α thienyl-4 thiazolyl-2) oxamate d'éthyle

Dans une solution de 36,5 g (0,2 mole) d'α thienyl-4 amino-2 thiazole dans 350 $cm^3$ de tétrahydrofu-

ranne et 56,6 $cm^3$ (0,4 mole) de triéthylamine, on ajoute goutte à goutte sous forme agitation une solution de 26,6 $cm^3$ (0,22 mole) de chlorure d'oxalate d'éthyle dans 150 $cm^3$ de tétrahydrofuranne. Laisser une nuit sous agitation à température ambiante puis le solvant est évaporé sous pression réduite jusqu'à siccité.

Le résidu est lavé à l'eau plusieurs fois jusqu'à neutralité, puis recristallisé dans un mélange acétate d'éthyle-éther isopropylique.

On récupère 41,5 g de produit (rendement : 73,5 %) de formule :

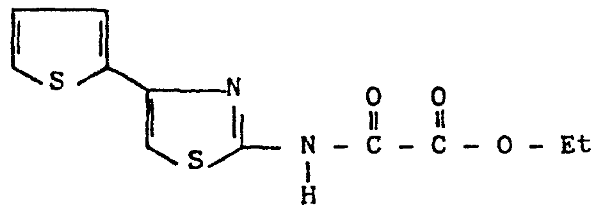

Formule brute : $C_{11}H_{10}N_2O_3S_2$.
Masse moléculaire : 282,3.
Cristaux : jaunes.
Point de fusion : 149 °C.
Chromatographie sur plaque :
— support : gel de silice 60 F 254 Merck
— solvant : acétate d'éthyle-éther de pétrole 50/50
— révélation : UV et iode
— Rf : 0,6.
Solubilités : insoluble dans l'eau, soluble à 1 % dans l'éthanol et à 50 % dans la méthyl pyrrolidone.

Exemple 2

Acide (α thienyl-4 thiazolyl-2) oxamique

A une suspension de 28 g d'(α thienyl-4 thiazolyl-2) oxamate d'éthyle dans 560 $cm^3$ d'eau, on ajoute 110 $cm^3$ de soude (N), puis ce mélange réactionnel est chauffé à ébullition 30 minutes jusqu'à dissolution.

Par acidification avec de l'acide chlorhydrique 2 N on obtient un précipité qui est séparé par filtration. Le résidu est mis deux fois en suspension dans l'eau, filtré et lavé à l'éthanol.

L'acide est purifié par recristallisation dans un mélange diméthyl formamide éthanol. On récupère avec un rendement de 82 % le produit de formule :

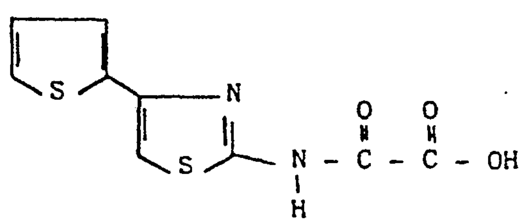

Formule brute : $C_9H_6N_2O_3S_2$.
Masse moléculaire : 254,3.
Cristaux : beiges.
Point de fusion : 250 °C (décomposition).
Chromatographie sur plaque :
— support : gel de silice 60 F 254 Merck
— solvant : chloroforme-méthanol-ammoniaque 70/35/10
— révélation : UV et iode
— Rf : 0,27.
Solubilités : insoluble dans l'eau et soluble à 50 % dans la méthyl pyrrolidone.

Exemple 3

(α thienyl-4 thiazolyl-2) oxamate de sodium

Dissoudre 10,17 g d'acide (α thienyl-4 thiazolyle-2) oxamique dans 100 $cm^3$ de dioxanne et ajouter 40 $cm^3$ de soude (N).

A cette solution l'on ajoute lentement 250 cm³ d'acétone, le sel de sodium précipite, on récupère après filtration et séchage 8,55 g de sel de sodium (77 %) de formule :

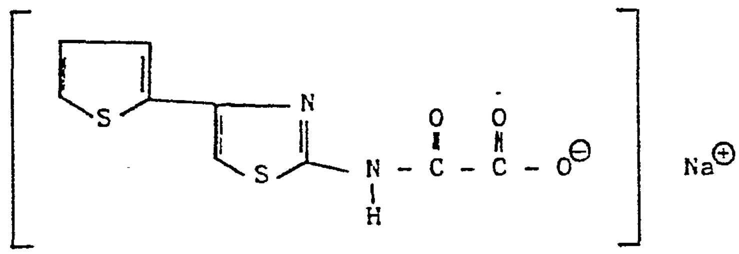

Formule brute : $C_9H_5NaN_2O_3S_2$.
Masse moléculaire : 276,2.
Cristaux : beiges.
Point de fusion : > à 300 °C.
Chromatographie sur plaque :
— support : gel de silice 60 F 254 Merck
— solvant : chloroforme-méthanol-ammoniaque 70/35/10
— révélation : UV et iode
— Rf : 0,27.
Solubilités : insoluble dans l'eau. Solution à 15 % dans la méthyl pyrrolidone.

Exemple 4

(α thienyl-4 thiazolyl-2) oxamate de zinc

A une solution de 4 g (α thienyl-4 thiazolyl-2) oxamate de sodium dans 200 cm³ d'eau chaude, ajouter 4,4 g de sulfate de zinc en solution dans 10 cm³ d'eau. Le précipité jaune formé est récupéré par filtration, lavé deux fois à l'eau. Après séchage, on récupère avec un rendement de 82 % 3,4 g de produit de formule :

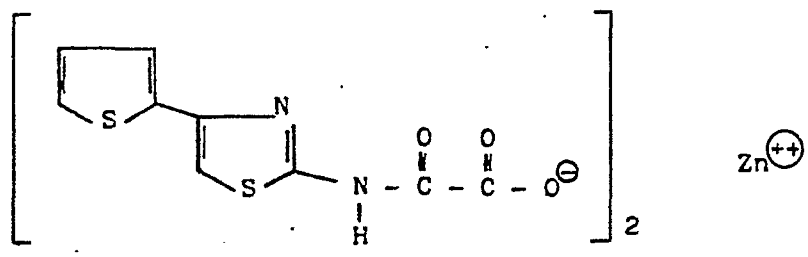

Formule brute : $C_{18}H_{10}N_4O_6S_4Zn, H_2O$.
Masse moléculaire : 589,9.
Cristaux : jaunes.
Point de fusion : > à 300 °C.
Chromatographie sur plaque :
— support : gel de silice 60 F 254 Merck
— solvant : chloroforme-méthanol-ammoniaque 70/35/10
— révélation : UV et iode
— Rf : 0,27.
Solubilités : insoluble dans l'eau. Soluble dans la méthyl pyrrolidone à 50 %.

Exemple 5

(α thienyl-4 thiazolyl-2) oxamate d'éthanolamine

Dissoudre à chaud 3,6 g d'acide (α thienyl-4 thiazolyl-2) oxamique dans 150 cm³ d'une solution 50/50 chloroforme-méthanol. Puis on ajoute à cette solution 1,8 cm³ d'éthanolamine.

La solution est concentrée, par addition d'éther éthylique on amorce la cristallisation. On récupère après filtration et séchage 4 g de produit (rendement 91 %) de formule :

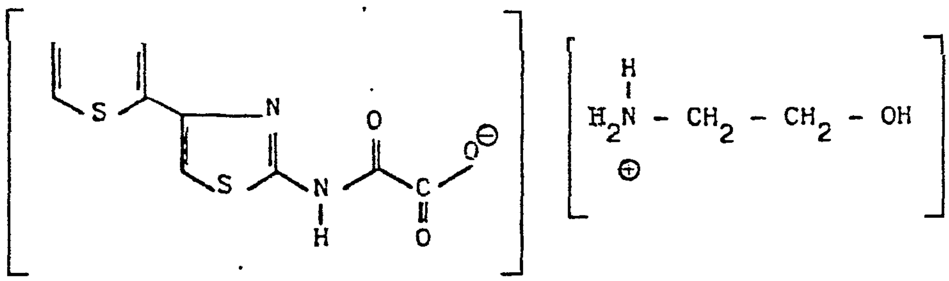

Formule brute : $C_{11}H_{13}N_3S_2O_4$.
Masse moléculaire : 315,3.
Cristaux : beiges.
Point de fusion : 184 °C.
Chromatographie sur plaque :
— support : gel de silice 60 F 254 Merck
— solvant : chloroforme-méthanol-ammoniaque 70/30/15
— révélation : UV et iode
— Rf : 0,62.
Solubilités : insoluble dans l'eau. Soluble à 15 % dans la méthyl pyrrolidone.

Exemple 6

(β thienyl-4 thiazolyl-2) oxamate d'éthyle

D'une façon similaire à celle décrite dans l'exemple 1, mais en utilisant l'acétyl-3 thiopène, on obtient le produit de formule :

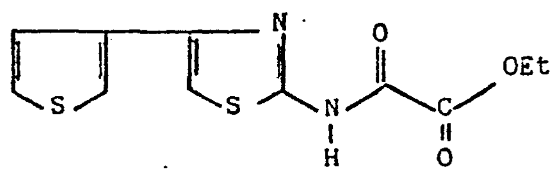

Formule brute : $C_{11}H_{10}N_2O_3S_2$.
Masse moléculaire : 282,3.
Cristaux : jaunes.
Point de fusion : 154 °C.
Chromatographie sur plaque :
— support : gel de silice 60 F 254 Merck
— solvant : acétate d'éthyle-éther de pétrole 50/50
— révélation : UV et iode
— Rf : 0,62.
Solubilités : insoluble dans l'eau. Soluble à 0,5 % dans l'éthanol et 50 % dans la méthyl pyrrolidone.

Exemple 7

(α furyl-4 thiazolyl-2) oxamate d'éthyle

D'une façon similaire à celle décrite dans l'exemple 1, mais en utilisant l'acétyl-2 furanne on obtient le produit de formule :

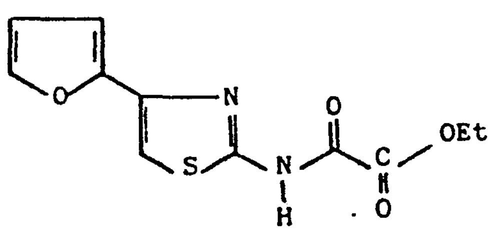

Formule brute : $C_{11}H_{10}N_2OS_4S$.
Masse moléculaire : 266,2.
Cristaux : jaunes.
Point de fusion : 127 °C.

Chromatographie sur plaque :
— support : gel de silice 60 F 254 Merck
— solvant : acétate d'éthyle-éther de pétrole 50/50
— révélation : UV et iode
— Rf : 0,58.
Solubilités : insoluble dans l'eau. Soluble à 3 % dans l'éthanol et à 50 % dans la méthyl pyrrolidone.

Exemple 8

Acide (α furyl-4 thiazolyl-2) oxamique

D'une façon similaire à celle décrite dans l'exemple 2, mais en utilisant l'(α furyl-4 thiazolyl-2) oxamate d'éthyle, on obtient le produit de formule :

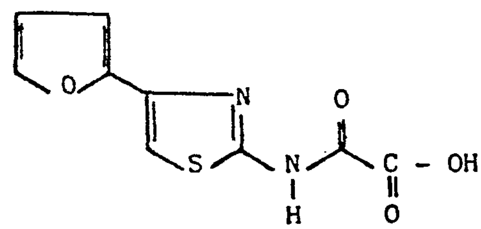

Formule brute : $C_9H_6N_2O_4S$.
Masse moléculaire : 238,2.
Cristaux : jaunes.
Point de fusion : > à 300 °C.
Chromatographie sur plaque :
— support : gel de silice 60 F 254 Merck
— solvant : chloroforme-méthanol-ammoniaque 70/35/10
— révélation : UV et iode
— Rf : 0,36.
Solubilités : insoluble dans l'eau. Soluble à 25 % dans la méthyl pyrrolidone.

Expérimentations

A) Toxicologie

Les composés chimiques précédemment décrits ont été soumis à des contrôles de toxicité.

L'étude de la toxicité a été effectuée chez la souris conventionnelle pesant de 20 à 22 grammes.

Les substances ont été administrées par voie orale. La DL 50 est calculée selon la méthode de MILLER et TAINTER (Proc. Soc. Exper. Biol. Med., 1944, 57, 261). Les DL 50 sont comprises entre 750 et 2 000 mg/kg.

B) Etude pharmacologique

Les résultats sont obtenus sur le test d'anaphylaxie passive cutanée selon la méthode de BROCKLEHURST W.E. in Handbook of Experimental Immunolog. Ed. Weiz D.M. Blackwell Scientific Publications, Oxford and Edimbourg, p. 745-751 (1967).

a) Préparer l'antisérum sur rats sensibilisés par l'antigène, l'injecter par voie intradermique.
b) Laisser un temps de latence de 24 à 48 heures.
c) Administrer le produit à tester
   per os 10 minutes avant l'opération d)
   i. v. simultanément à d)
d) Injection i. v. de l'antigène + colorant.
e) Sacrifier 30 minutes après et visualiser la réaction antigène anticorps par la fuite du colorant due à l'augmentation de la perméabilité capillaire sous l'influence combinée de l'histamine et du SRSA.

Cette action est exprimée en ED 50 dose qui réduit de moitié la fuite du colorant par rapport aux témoins.

Les ED 50 des composés les plus actifs sont de l'ordre de 5 mg/kg par voie orale.

Les essais ont été effectués successivement à 30, 5, 1 et 0,2 mg/kg sur des lots homogènes de 10 rats. Compte tenu de leur insolubilité dans l'eau, ces composés sont administrés en suspension dans le tween-eau.

C) Application thérapeutique

Compte tenu de leurs propriétés pharmacologiques et leur faible toxicité, ces composés sont utilisables par voie orale en préventif vis-à-vis des crises d'asthme allergique.

Ces composés peuvent être utiles également dans le traitement de l'urticaire, des rhinites allergiques et certaines allergies cutanées.

Les dosages auxquels les composés actifs peuvent être administrés peuvent varier dans des proportions importantes selon l'état du patient. Un dosage quotidien d'environ 5 à 500 mg/jour est toutefois préféré. Ces composés actifs sont utilisés sous forme de préparations pharmaceutiques adaptées facilitant la biodisponibilité.

Ces préparations peuvent se présenter sous forme solide par exemple de comprimés, dragées, capsules ou sous forme liquide par exemple solutions, suspensions ou émulsions.

**Revendications**

1. Dérivés de l'acide thiazolyl-2 oxamique substitués en position – 4 répondant à la formule générale I :

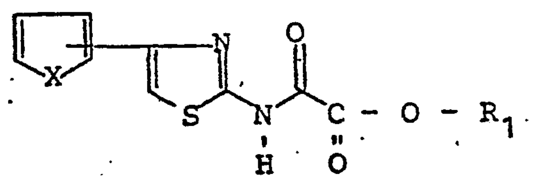

(I)

dans laquelle :

$R_1$ représente un atome d'hydrogène ou un radical alcoyle comprenant de 1 à 4 atomes de carbone et X représente un atome de soufre ou d'oxygène.

Ainsi que les sels avec les bases organiques ou minérales thérapeutiquement acceptables des composés dans lesquels $R_1$ représente un atome d'hydrogène.

2. Composés de formule générale I selon la revendication 1, caractérisés par le fait qu'ils sont choisis parmi :

— (α thienyl-4 thiazolyl-2) oxamate d'éthyle,
— Acide (α thienyl-4 thiazolyl-2) oxamique,
— (α thienyl-4 thiazolyl-2) oxamate de sodium,
— (α thienyl-4 thiazolyl-2) oxamate de zinc,
— (α thienyl-4 thiazolyl-2) oxamate d'éthanolamine,
— (β thienyl-4 thiazolyl-2) oxamate d'éthyle.

3. Composés de formule générale I selon la revendication 1, caractérisés par le fait qu'ils sont choisis parmi :

— (α furyl-4 thiazolyl-2) oxamate d'éthyle,
— acide (α furyl-4 thiazolyl-2) oxamique.

4. Procédé de préparation des composés de formule générale I, selon les revendications 1 à 3 caractérisé par le fait qu'il consiste à faire réagir un amino-2 thiazole de formule II :

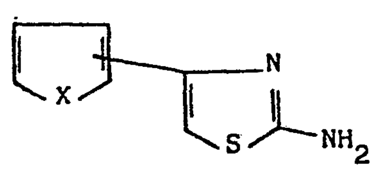

(II)

dans laquelle :

X représente un atome de soufre ou d'oxygène avec un halogènure d'ester de l'acide oxalique et en ce que l'on saponifie éventuellement l'ester ainsi obtenu et si on le désire on transforme l'acide obtenu en un sel avec une base minérale ou organique thérapeutiquement acceptable.

5. A titre de médicaments nouveaux utiles notamment au traitement de l'asthme allergique, de l'urticaire et des eczémas, les composés selon l'une des revendications 1 à 3.

6. Compositions pharmaceutiques caractérisées par le fait qu'elles contiennent à titre de substance active au moins un composé selon les revendications 1 à 3.

7. Compositions pharmaceutiques selon la revendication 5 caractérisées en ce que d'autres

principes actifs sont associés aux composés de formule générale I selon les revendications 1 à 3 pour compléter ou renforcer leur actions thérapeutiques.

**Claims**

1. 2-thiazolyloxamic acid derivatives that are substituted in the 4-position and correspond to the general formula I :

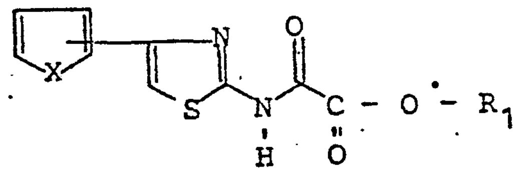

(I)

in which :

$R_1$ represents a hydrogen atom or an alkyl radical containing from 1 to 4 carbon atoms, and

X represents a sulphur atom or an oxygen atom, and the salts with therapeutically acceptable organic or mineral bases of the compounds in which $R_1$ represents a hydrogen atom.

2. Compounds of the general formula I according to claim 1, characterised by the fact that they are selected from :

— ethyl (4-(α-thienyl)-2-thiazolyl)-oxamate,
— (4-(α-thienyl)-2-thiazolyl)-oxamic acid,
— sodium (4-(α-thienyl)-2-thiazolyl)-oxamate,
— zinc (4-(α-thienyl)-2-thiazolyl)-oxamate,
— ethanolamine (4-(α-thienyl)-2-thiazolyl)-oxamate, and
— ethyl (4-(β-thienyl)-2-thiazolyl)-oxamate.

3. Compounds of the general formula I according to claim 1, characterised by the fact that they are selected from :

— ethyl (4-(α-furyl)-2-thiazolyl)-oxamate and
— (4-(α-furyl)-2-thiazolyl)-oxamic acid.

4. Process for the preparation of the compounds of the general formula I according to claims 1 to 3, characterised by the fact that it comprises reacting a 2-aminothiazole of the formula II :

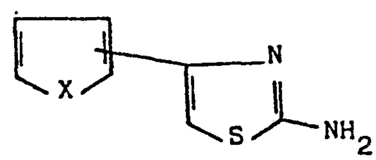

(II)

in which :

X represents a sulphur atom or an oxygen atom with an oxalic acid ester halide, and in that the ester so obtained is optionally hydrolysed and, if desired, the resulting acid is converted into a salt with a therapeutically acceptable mineral or organic base.

5. The compounds according to one of claims 1 to 3 as novel medicaments useful, especially, for the treatment of allergic asthma, urticaria and eczema.

6. Pharmaceutical compositions characterised by the fact that they contain as active ingredient at least one compound according to claims 1 to 3.

7. Pharmaceutical compositions according to claim 5, characterised in that other active ingredients are associated with the compounds of the general formula I according to claims 1 to 3 in order to supplement or reinforce their therapeutic actions.

**Ansprüche**

1. In der 4-Stellung substituierte 2-Thiazolyl-oxaminsäurederivate der allgemeinen Formel I :

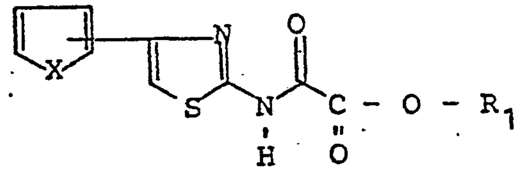

(I)

in der :

$R_1$ ein Wasserstoffatom oder eine Alkylgruppe mit 1 bis 4 Kohlenstoffatomen und

X ein Schwefelatom oder ein Sauerstoffatom bedeuten, sowie die Salze der Verbindungen, in denen $R_1$ ein Wasserstoffatom darstellt, mit organischen oder anorganischen, therapeutisch verträglichen Basen.

2. Verbindungen der allgemeinen Formel gemäß Anspruch 1, dadurch gekennzeichnet, daß sie ausgewählt sind aus :

— (4α-Thienyl-2-thiazolyl)-oxaminsäureethylester,
— (4α-Thienyl-2-thiazolyl)-oxaminsäure,
— (4α-Thienyl-2-thiazolyl)-oxaminsäure-natriumsalz,
— (4α-Thienyl-2-thiazolyl)-oxaminsäure-zinksalz,
— (4α-Thienyl-2-thiazolyl)-oxaminsäure-ethanolaminsalz,
— (4β-Thienyl-2-thiazolyl)-oxaminsäureethylester.

3. Verbindungen der allgemeinen Formel gemäß Anspruch 1, dadurch gekennzeichnet, daß sie ausgewählt sind aus :

— (4α-Furyl-2-thiazolyl)-oxaminsäureethylester,
— (4α-Furyl-2-thiazolyl)-oxaminsäure.

4. Verfahren zur Herstellung der Verbindungen der allgemeinen Formel I nach den Ansprüchen 1 bis 3, dadurch gekennzeichnet, daß man ein 2-Amino-thiazol der Formel II :

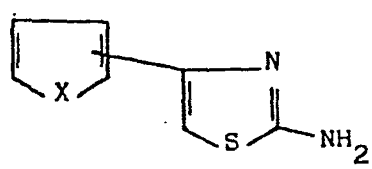

(II)

in der :

X ein Schwefelatom oder ein Sauerstoffatom bedeutet, mit einem Halogenid des Esters der Oxalsäure umsetzt, gegebenenfalls den in dieser Weise erhaltenen Ester verseift und gewünschtenfalls die erhaltene Säure mit einer anorganischen oder organischen therapeutisch verträglichen Base in ein Salz überführt.

5. Verbindungen nach einem der Ansprüche 1 bis 3 als Arzneimittelwirkstoffe insbesondere für die Behandlung von allergischem Asthma, Nesselfieber und Ekzemen.

6. Pharmazeutische Zubereitungen, dadurch gekennzeichnet, daß sie als Wirkstoff mindestens eine Verbindung nach den Ansprüchen 1 bis 3 enthalten.

7. Pharmazeutische Zubereitungen nach Anspruch 5, dadurch gekennzeichnet, daß neben den Verbindungen der allgemeinen Formel I gemäß den Ansprüchen 1 bis 3 weitere Wirkstoffe zur Ergänzung oder Verstärkung ihrer therapeutischen Wirkungen vorhanden sind.